# EUROPEAN PATENT SPECIFICATION

(11) **EP 1 609 441 B1**
(45) Date of publication and mention of the grant of the patent: **01.07.2009**
(21) Application number: 03785961.8
(22) Date of filing: 16.12.2003
(51) Int. Cl.: A61F 2/28, A61L 27/10

(54) **BIOCOMPATIBLE MATERIAL**
BIOKOMPATIBLES MATERIAL
MATERIAU BIOCOMPATIBLE

(30) Priority: 20.12.2002 ES 200203052
(43) Date of publication of application: 28.12.2005
(73) Proprietor: Universidad de Sevilla,, 41013 Sevilla (ES); Universidad de Vigo, 36200 Vigo (Pontevedra) (ES)
(72) Inventor: Martinez Fernandez, Julián, 41012 Sevilla (ES); Ramirez de Arellano Lopez, Antonio, 41012 Sevilla (ES); Varela Feria, Francisco Manuel, 41012 Sevilla (ES); Gonzalez Fernandez, Pio Manuel, 36200 Vigo (Pontevedra) (ES); Serra Rodriguez, Julia A., 36200 Vigo (Pontevedra) (ES); Liste Carmueja, Sara, 36200 Vigo (Pontevedra) (ES); Chiussi, Stefano, 36200 Vigo (Pontevedra) (ES); Perez Borrajo, Jacinto, 36200 Vigo (Pontevedra) (ES); Arias Otero, José Luis, 36200 Vigo (Pontevedra) (ES); Leon Fong, Betty, 36200 Vigo (Pontevedra) (ES); Perez-Martinez Y Perez-Amor, Mariano, 36200 Vigo (Pontevedra) (ES)
(74) Representative: Temino Ceniceros, Ignacio
(86) International application number: PCT/ES2003/000638
(87) International publication number: WO 2004/056292

(56) References cited:
- WO-A-96/21628
- SERRA J. ET AL.: 'Processing of bioglass coatings by excimer lase ablation' KEY ENGINEERING MATERIALS vol. 192, 2001, pages 635 - 638, XP001223530
- D'ALESSIO L. ET AL.: 'Pulsed laser ablation an deposition of bioactive glass as coating material for biomedical applications' APPL. SUR. SCIENCE vol. 138-139, 1999, pages 527 - 532, XP002992595
- MARTINEZ-FERNANDEZ J. ET AL.: "High temperature compressive mechanical behavior of biomorphic silicon carbide ceramics" SCRIPTA MATERIALIA, vol. 43, 2000, pages 813-818,

## Description

### OBJECT OF THE INVENTION

The invention relates to a novel process for the production of a biocompatible material which is intended to be used in the production of implants, prostheses or biomedical devices, comprising biomorphic SiC ceramics as a support material with a coating of bioactive glass that is deposited by means of pulsed laser ablation.

The novel aspect of this invention lies in the joint use of biomorphic ceramic materials and the bioactive glass coating. The main advantage that it has when compared to other products or biomedical devices, is the combination of a suitable bioactivity that gives it a bioactive glass coating that possesses the mechanical advantages that are provided by an inert support with a porous texture (biomorphic SiC ceramics), thereby achieving a bioactive setting between the implant and the bone.

The specific advantages that are offered by the joint use of biomorphic SiC ceramics and the bioactive glass coating that is deposited by means of pulsed laser ablation are as follows:
a) A density that is much lower than the density of implants that are manufactured with other materials, for example titanium.
b) Low cost, due to the low price of the components and the fact that little final machining is necessary.
c) High mechanical strength.
d) A wide range of microstructures and properties in the substrata can be obtained, which can be used for specific applications.
e) A deposit of adhesive coatings of bioactive glass that can excellently cover the porous texture of the biomorphic ceramics.
f) A suitable bioactivity for these coatings. It has been demonstrated by conducting *in vitro* tests, in which the coatings have been submerged in simulated body fluid, that a process takes place in which there is a reprecipitation of a layer of calcium phosphate that is similar to that of bones. This layer is formed more rapidly when the substratum that is used is made of biomorphic ceramics than when the materials are completely dense.
g) It is highly flexible and enables the user to vary the physicochemical properties of the coatings by merely modifying the processing conditions. This aspect is of great importance in view of the fact that it makes it possible to control at will the bioactivity ratio so that the most suitable ratio can be found for each specific application. It is thus possible to design coatings with optimum characteristics depending upon the type of tissue (cortical, trabicular, etc.) with which the biomedical implant is going to interact.
h) It has several advantages over other depositing techniques, such as the absence of high temperatures, no contamination of the coating because the process takes place in vacuum and there is an excellent control over the phases and the physicochemical properties of the coating due to suitable bioactivity of the material.
i) This method makes it possible to coat pieces of biomorphic ceramics already completed without any subsequent machining, in view of the fact that during the depositing process their size is not altered, in such a way that the coated pieces will carry on complying with the same tolerance levels as they did in the first place.

### STATE OF THE ART

### Introduction

The major developments that have been made in the Science and Technology of Materials in the last thirty years, have yielded a wide range of possibilities when it comes to choosing the material that will provide the requirements of a particular application. To be specific, in the world of biomaterials we can mention the development of metallic materials (stainless steel, Co-Cr, titanium and alloys), ceramic materials (alumina, zirconia, graphite), polymeric materials (epoxies, Polymethyl Methacrylate [PMMA], Teflon) and composites, all of which are oriented towards the manufacture of implants, prostheses and medical devices that make it possible to repair or replace parts and/or functions of the human body that have been damaged [1]. The major developments that have been made in the field of implantology are due, to a large extent, to the improvement in the physicochemical properties and mechanical benefits of the different materials depending on the biofunctionality that is required. At present, the manufacture of implants for bone replacements is mainly based upon either the use of pure titanium or alloys (Ti6A14V, Ti-5A1-2.5E or Ti6A17Nb), with or without coatings, and this technology is moving towards the development of new products that are lighter, more resistant to wear and tear and ones that offer a better biological response.

The materials that are implanted in a living tissue cause a biological response from the latter, at the interface between the implant and the tissue. This response can either be toxic in nature, bringing about the death of the tissue, or the implanted material can be biocompatible, in which case the response can be inert, reabsorbable or bioactive.

Therefore, we can establish the following classification for biocompatible materials:
a) Inert biomaterials: these materials are accepted by the body because they are biologically inert and they can also withstand long periods in a highly corrosive environment, such as one in which there are body fluids. The drawback to these materials is that there is invariably a small gap at the interface between the implant and the host tissue, which gives rise to the formation of a thin, non-adhesive, fibrous capsule around the implant. Bone might be formed on the surface but there is no link between the bioinert material and the host tissue. Titanium, chrome cobalt and their alloys belong to this group of materials. Their mechanical properties (fatigue stress, resistance to corrosion and plastic deformation) are suitable for enabling them to be used as permanent prostheses. The use of stainless steel (medical grade) is reserved for temporary implants because their resistance to corrosion is poor. The use of alumina and zirconia (A1203 and ZrO2) is also limited, due to the fact that their resistance to rupture is poor (bending-induced rupture) although they are very stable oxides with an excellent resistance to friction and corrosion. However, they are excellent materials for certain parts of a prosthesis where low friction and compressive stress are required, as is the case at the head of a hip replacement prosthesis.
b) Inert biomorphic materials
   The use of natural precursors in the manufacturing process for ceramics is an extremely novel technique that has been patented by the University of Seville [2].
   For some considerable time scientists working on materials have been trying to obtain ceramics that blend with natural structures, and these have been perfected for specific services by natural evolution. A wide variety of procedures have been adopted for achieving this aim, and they have generally been expensive, because an attempt has been made to "manufacture" the microstructure. The use of wood to manufacture biomorphic silicon carbide is a highly novel idea and one that has great potential. By means of a process that has been patented by the University of Seville, the microstructure of wood is conserved and its good mechanical properties are "enhanced" on being transformed into silicon carbide, using a cheap process that can be optimised on the basis of the applications concerned.
   The results obtained by the University of Seville [3-13] indicate that these ceramics have the following advantages over other procedures that are used for manufacturing ceramics:
   1. Low cost (less than all the earlier techniques used) due to the following:
      - The processing takes place at a temperature of 1,410 °C, which is between 600 and 900 °C less than when sintering is used.
      - Unlike the sintering and reaction process, it does not need to start from silicon carbide powder. All that is required is the vegetable precursor and silicon, both of which cost very little.
      - The pieces do not need a final finish (if they are processed in accordance with the instructions that are given in the patent taken out by the University of Seville).
   2. The use of materials that can be regenerated. They do not contaminate the environment.
   3. The potential for manufacturing complex forms merely by modelling the source timber beforehand (simpler and more economical than all the earlier processing procedures).
   4. No additives are required.
   5. The manufacturing process takes place more quickly and at a lower temperature than the manufacturing procedures involving reaction with gases.

   The ceramics that are obtained have the same fibrous structure as the wood that was used in the manufacturing process, this being an ideal structure for obtaining optimum mechanical properties in view of the fact that it is a result of perfecting the process of evolution. Strengths that are similar to sintered SiC and much greater than SiC compacted by reaction [14-16] are obtained with densities that are 50% less.
   A structure that is similar to that of materials composed of continuous fibre [17] is obtained naturally. These materials are designed to improve the low intrinsic toughness of silicon carbide.
   It is possible to obtain a wide range of microstructures and properties for specific applications merely by selecting the right vegetable precursor.
   In view of their excellent properties, these materials can be used in applications that require great strength, low density, controlled porosity and/or a high specific surface, especially low density structures for medical implants.
c) Biodegradable biomaterials: these materials are designed to degrade gradually and to be replaced by the host tissue. They are used as fill materials and are an optimum solution when it is possible to cure the tissue itself in the short term. The drawback lies in the fact that the constituents of the degraded material must be accepted physiologically and it is difficult to adjust the biodegradation ratio of the material and the ratio of formation for the new tissue. The first application of this type is reabsorbable stitching (polyglycolic acid). A variety of polymers and ceramics, such as calcium salts, are among these materials.
d) Bioactive biomaterials; this term refers to biomaterials that are designed to have a modulated biological activity. When they are exposed to body fluids, bioactive materials react chemically to form an interfacial link between the implant and the host tissue (bone tissue or soft tissue). Depending on their composition, bioactive materials can either act as osteoconductors, such as hydroxyapatite, or as an osteopromotor, that is to say, inducing the formation of new bone, as is the case with bioactive glass and some vitreous ceramics (Ceravital, Bioverit, Limaplant). This subject will be dealt with in greater depth in a subsequent section.

One aspect that is vital to implantology is to manage to get a stable mechanical anchorage for the implants or permanent devices that have to be incorporated into the body and fixed there. Basically, the attachment methods can be split into two groups:
a) Cemented fixation: where the implant is stuck to the host bone with the aid of an acrylic bone cement. This technical solution was introduced in the 1970s, and it was the most suitable and most widespread technique used for elderly patients whose bone quality was poor. There are several problems due to failures associated with the acrylic cement (setting, fragmentation, breakage) when applied, because if the cement coating around the stem of the implant is incomplete, this can lead to a weakening of the prosthesis.
b) Uncemented fixation: at present the uncemented fixation concept has been opted for, especially in the case of young patients who are highly active from a physical perspective. With a view to this, methods and materials have been developed that improve the interfacial stability between the implant and the host tissue. In the latter group, the implant can be fixed orthopaedically in any of the following ways:
   1) Morphological fixation, where the implant is inserted into a bony bed. Pieces of titanium with microroughness on the surface are generally used because this is conducive to stabilising the implant.
   2) Biological fixation, where the implant is held in place thanks to the growth of the bone tissue between the pore interstices or textured surface of the implant. Histological studies have shown that the growth of the bone between the pores of the implant is less than was hoped for due to the inert nature of the material that has been implanted, because it does not stimulate bone growth.
   3) Bioactive fixation, where the implant is bonded to the bone by means of chemical processes that occur on the surface of the implant. This type of fixation amounts to a considerable qualitative leap forward because it provides a genuine chemical bond between the implant and the tissue. The synthetic materials that cause this response are defined as bioactive materials and they are used as filler material or are applied as coatings on metals, polymers or ceramic materials, given that these do not support an excessive load.

### Coatings of bioactive glass

One novel aspect of biomaterial technology is the coating of dental implants and orthopaedic prostheses by means of fine layers of latest generation bioactive glass. The great interest that bioactive materials have aroused lies in their capacity to induce or enhance the formation of new bone and even soft tissues [1, 18]. Coating implants with a thin layer of bioactive glass has major advantages: first of all, it leads to the benefit of securing the bone better (bioactive anchorage ), in view of the fact that new bone tissue is formed and, furthermore, it will act as a barrier that prevents the elements from dissipating from the base material of the prosthesis, while at the same time protecting the implant against corrosion due to the aggressive environment of the body fluid; this serves to overcome certain problems that exist with current implants, namely the lack of bonding between the material that has been implanted and the host tissue and the formation of a fibrous capsule around the implant.

Document WO 96/21628 describes bioactive glasses that may be used as coatings on e.g. alloys, metals, other glasses and ceramics.

Bioactive glass, within the system SiO₂-Na₂O-CaO-P₂O₅, makes available a great variety of materials with different degrees of bioactivity depending on their composition. By way of example, we could mention that glass with a content of more than 60% SiO₂ has no bioactive properties whatsoever and, by contrast, a high content of Na₂O or CaO increases the reactivity nature of the material.

As yet, a full in-depth working knowledge of the chemical mechanisms that govern the bioactivity of these materials has not been obtained. These bioactive glasses dissolve in the presence of the body fluids, forming silanol groups (SiOH) that on reaction give rise to a silica gel (SiO₂) on the surface of the glass. While the glass is in the process of dissolving ions of Ca and P are released, these being present in the glass composition in a high ratio (CaO / P₂O₅, which give rise to the formation of an amorphous layer of calcium phosphate (a-CaP) on the SiO₂ gel. The alkaline pH of the glass solution interface causes the a-CaP layer to crystallise into a hydrocarbonate apatite (CHA) that easily bonds with the bony tissue [1, 19, 20 and 21].

These materials have recently been perfected, and bioactive glasses of the latest generation have been developed in the SiO₂-Na₂O-K₂O-MgO-B₂O₃-CaO-P₂O₅ system that have an optimised degree of bioactivity. Their chemical constituents lie somewhere between the physiological compounds of the body (Si, Na, K, Mg, O, Ca and P) without the levels of concentration being able to alter the nearby tissues. These bioactive glasses have been successfully used as filler material and porous bodies have also been manufactured by sintering microspheres. The *in vitro* and *in vivo* tests have demonstrated that these materials bring about the growth of bone tissue [18, 22 and 23].

Furthermore, it has also been shown that bioactive glass can be applied as a coating in thin layers of metallic implants using a wide variety of deposition methods, such as enamelling, rapid immersion, flame spray and plasma spray. More recently, laser techniques have been successfully applied (pulsed laser deposition PLD) to obtain coatings of these materials [24-26], which overcome some of the deficiencies and drawbacks involved in the other techniques (a detailed account of these methods is given in the next section).

Document XP-001223530 describes the processing of bioglass coatings by excimer laser ablation.

By way of a summary, a new generation of implants must combine the suitable bioactivity that gives it the bioactive glass coating with the mechanical advantages that offer an inert support with a porous texture (biomorphic ceramics of SiC), thereby achieving a bioactive fixation between the implant and the bone.

### Methods for depositing bioactive glass

The most frequently used processing methods for coating dental implants and metallic orthopaedic implants (stainless steel 316L, titanium alloys T16A14V and of Co-Cr-Mo) by means of fine layers of bioactive glass are as follows:
a) Enamelling: this is the traditional method for applying a coating of glass to a metal. The process consists of covering the metal with an aqueous mixture of the powder and letting it dry; then the coated metal is heated to temperatures ranging from 400 to 600 °C so that the glass melts into a layer of oxide on the surface of the metal. Adherence is improved by using a base layer that is applied between the substrate and the bioactive glass [27, 28].
b) Rapid immersion: in which the metal is pre-oxidised by subjecting it to a certain temperature and, then, submerging it in a container with melted bioactive glass and keeping it at a high temperature (1,300 °C). The layer of oxide dissolves to form an adhesive bond between the substrate and the glass coating [1].
c) Flame spray: with this method the bioactive glass in powdered form is made to pass through a hydrogen-oxygen flame at a temperature of 3,000 °C, so the powder heats up rapidly and melts; the jet of melted particles is projected to the substrate and when it cools it forms a layer of glass. To prevent bubbles from forming some substitutes of B₂O₃ and CaF₂ to modify the way in which the viscosity behaves with the temperature [1].
d) Plasma spray: this is a variation of the preceding method, in which a direct current (DC) electric charge is caused in a gas and bringing about a high temperature and velocity plasma [28-30]. The powdered glass is injected into this plasma causing the particles to melt and they are expelled towards the substrate. These are the techniques that are most often used commercially and they enable the user to produce thick coatings (80 microns). This thickness is an intermediate between the minimum thickness and the maximum homogeneity that is permitted by this method of deposition.

### Problems in manufacturing medical implants that have been solved by this Patent

### A. Inert Biomaterials

Biomorphic ceramics have the following advantages over the usual inert biomaterials:
a. Lower density, close to that of the bone, which is very important when it comes to improving the integration of the implant and causing less collateral damage to the patient.
b. A lower cost, due to the materials that are used and the less final finish involved.
c. A porous structure that enhances the formation of a layer of calcium phosphate whose composition is similar to that of the bone, which is conducive to biocompatibility.

### B. Bioactive coating

Up to the present time, the use of commercial coatings of bioactive glass has been very limited, owing to deficiencies and technological problems affecting these methods that have yet to be overcome:
a. The high temperatures required to melt the material, which can have an adverse effect upon the substrate and prevent the coating on thermosensitive supports ( polymeric materials ) or the inclusion of layers of biomolecules;
b. Reliability of the adhesion between the metal and the coating, due to the different coefficients of heat expansion between glass and metal;
c. The formation of cracks and open paths in the coating, which appear during the course of the cooling process, and which enable the metallic ions (T, Fe, Cr, etc. ) to migrate from the substrate to the coating and prevent bioactivity. Furthermore, the metallic substrate comes into contact with the biological fluids, which causes the corrosion of the former;
d. The substitutes introduced in the bioactive glass to prevent the formation of bubbles prevents the soft connective tissue from taking a hold;
e. Unpredictable phase changes in the particles of melted glass.

One method for producing coatings that serves as an alternative to those that have already been mentioned is the technique referred to as the pulsed laser deposition (PLD) technique [24-26, 31-32]. It is based upon the use of a pulsed excimer laser beam that ablates a target of material to be deposited giving rise to the formation of a plasma that is known as an ablation plume, which is projected onto the substrate that has to be coated. The control over the parameters that take part in the processing of these thin deposits (the composition of the target, the temperature of the substrate, the distance from the substrate to the target, the laser energy, etc.) affect its morphology and its physicochemical properties.

The preliminary results that have been obtained [21, 32] indicate that this technique has several advantages and manages to overcome the technological problems that the other depositing techniques are fraught with:
a. No high temperatures are involved in the process;
b. The stequiometry of the target material is transferred from the target to the substrate;
c. Good control over the physicochemical properties of the coatings;
d. As the process takes place in a vacuum there is no contamination.

### References

1. "An Introduction to Bioceramics", Ed. L. Hench and J. Wilson, World Scientific, 1993.
   X. Liu, S. Tao, C. Ding: "Bioactivity of plasma sprayed dicalcium silicate coatings", Biomaterials 23 (2002) 963.
2. J. Martinez Fernandez, A. R. De Arellano López, F. M. Valera-Feria and M. Singh. "Procedure for the manufacture of silicon carbide from vegetable precursors" Patent P200102278 being dealt with, Spain 2001. International extension applied for on 14th November 2002.
3. J. Martínez Fernandez, F. M. Valera-Feria and M. Singh "High temperature compressive mechanical behaviour of biomorphic silicon carbide ceramics", Scripta Materialia 43 (2000) 813 - 818.
4. J. Martinez Fernandez, A. Muñoz, F. M. Valera-Feria and M. Singh, "Interfacial and Thermomechanical Characterization of Reaction Formed Joints in Silicon Carbide-Based Materials". J Eur. Ceram. Soc., 20 (2000) 2641 - 2648.
5. F. M. Valera-Feria, S. López Pombero, J. Martínez-Fernández, A. Ramirez de Arellano López and M. Singh, "Creep Resistant Biomorphic Silicon Carbide-Based Ceramics", Ceramics Engineering Science Proceedings Vol 22 [ 3 ], pp (2001) 135 - 145.
6. F. M. Valera-Feria, J. Martínez-Fernández, A. Ramírez de Arellano López and M. Singh, "Precursors Selection for Property Optimization in Biomorphic SiC Ceramics". Accepted for publication in Engineering Science Proceedings Vol 23 [ 4 ] pp. 681 - 685 (2002).
7. F. M. Valera-Feria, A. Rarmírez de Arellano López and J. Martinez-Fernández, "Ceramic Wood: Manufacture and Properties of Biomorphic Silicon Carbide", Bol. Soc. Esp. Ceram. y Vidrio 41 [4 ] 377 - 384 (2002).
8. F. M. Valera-Feria, J. Martínez-Fernández, A. Ramírez de Arellano López and M. Singh, "Low Density Biomorphic Silicon Carbide: Microstructure and Mechanical Properties" J. Europ. Ceram. Soc. Vol. 22 [ 14-15 ] pp. 2719 - 2725 (2002).
9. J. Martínez-Fernández, F. M. Valera-Feria, A. Dominguez Rodriguez and M. Singh "Microstructure and Mechanical Behaviour of Silicon Carbide Biomorphic Ceramics at High Temperatures". Revista de Metalurgia de Madrid Vol. 37 (2001) 295 - 298.
10. R. Sepúlveda, F. M. Valera-Feria, J. Martínez-Fernández, A. Ramírez de Arellano López. "Microstructure and Mechanical Properties of Biomorphic SiC Made from Gum Cistus Wood". Mechanical Properties of Solids, ISBN 84 - 9705 - 190 - 4, pp. 899 - 904 (2002).
11. Ma Esther Enrique Magarino, A. R. de Arellano López, J. Martinez-Fernández, K. C. Goretta "Mechanical Properties of Porous Silicon Carbide with an Open Cellular Structure". Mechanical Properties of Solids, ISBN 84 - 9705 - 190 - 4, pp. 699 - 704 (2002).
12. J. Martínez-Fernández, F. M. Valera-Feria, A. Dominguez Rodriguez and M. Singh, "Microstructure and Thermomechanical Characterisation of Biomorphic Silicon Carbide-Based Ceramics, Environment Conscious Materials; Ecomaterials. ISBN: 1 - 894475 - 04 - 6. Canadian Institute of Mining, Metallurgy and Petroleum, pp. 733 - 740 (2000).
13. A. Munoz, J. Martínez-Fernández, A. R. Pinto Gómez, and M. Singh "Microstructure and High Temperature Compressive Mechanical Behaviour of Joints in Biomorphic Silicon Carbide Ceramics "Joining of Advanced and Speciality Materials III. ASM International, materials Park, OH, United States, pp. 7 - 14 (2002).
14. Sheldon M. Weiderhorn, B. J. Hockey and J. D. French, "Mechanisms of Deformation of Silicon Nitride at High Temperatures", J. European Ceramic Society, Volume 19, Issues 13 - 14, 2273 - 2284, 1999.
15. B. John and J. Wachtman, "Structural Ceramics" Vol. 29, Academic Press, 91 - 163, 1989.
16. Hockey, B. J., and Wiederhorn, S. M., "Effect of Microstructure on the Creep of Siliconized Silicon Carbide", J. Am. Ceram. Soc. 75 [ 7 ] 1822 - 30 (1992).
17. R. Naslain, "Materials Design and Processing of High Temperature Matrix Composites: State of the Art and Future Trends", Adv. Composite Mater. Vol. 8, N° 1, pp. 3 - 16, 1999.
18. H. O. Ylänen, T. Helminen, A. Helminen, J. Rantakokko, K. H. Karlsson, H. T. Aro; "Porous Bioactive Glass Matrix in Reconstruction of Articular Osteochondral Defects" Annales Chirurgiae et Gynaecologiae 88 (1999) 237.
19. O. H. Andersson, K. H. Karlsson; "On the Bioactivity of Silicate Glass", Journal of Non-Crystalline Solids 129 (1991) 145.
20. G. Ohtsuki, T. Kokubo and T. Yamamuro, "Mechanism of Apatite Formation on CaO-SiO2-P2O5 glasses in a simulated body fluid", Journal of Non-Crystalline Solids 143 (1992) 84.
21. O. Peitl, E. D. Zanotto, L. L. Hench; "Highly Bioactive P2O5-Na2O-CaO-SiO2 Glass Ceramics. Journal of Non-Crystalline Solids 292 (2001) 115.
22. H. O. Ylänen, C. Ekholm, N. Beliaev, K. H. Karlsson, H. T. Aro; "Comparison of Three Methods in Evaluation of Bone Ingrowth into Porous Bioactive Glass and Titanium Implants", Key Engineering Materials", Key Engineering Materials 192 - 195 ( 2001 ) 613.
23. K. Aitasalo, M. Peltola, J. Suonpää and A. Yli-Urpo; "Bioactive Glass S53P4 in frontal sinus obliteration: a 9-Year Experience", Key Engineering Materials 192 - 195 ( 2001 ) 877.
24. L. D'Alessio, R. Teghil, M. Zaccagnino, I. Zaccardo, D. Ferro, V. Marotta, "Pulsed Laser Ablation and Deposition of Bioactive Glass as Coating Material for Biomedical Applications". Appl. Surf. Aci. 138 - 139 (1999) 527.
25. R. Teghil, D'Alessio, D. Ferro, S. M. Barinov; "Hardness of Bioactive Glass Film Deposited on Titanium Alloy by Pulsed Laser Ablation", Journal of Materials Science Letters 21 (2002) 379.
26. J. Serra, P. González, S. Chiussi, B. León, M. Pérez Amor, "Processing of Bioglass Coatings by Excimer Laser Ablation", Key Engineering Materials 192 (2001) 635.
27. J. M. Gómez Vega, E. Saíz, A. P. Tomsia, G. W. Marshall, S. J. Marshall, "Bioactive Glass Coatings with hydroxyapatite and bioglass particles on Ti-based implants. Processing", Biomaterials 21 ( 2002 ) 105.
28. T. Oku, K. Suganuma, L. R. Wallenberg, A. P. Tomsia, J. M. Gómez-Vega, E. Saiz, "Structural Characterization of the Metal / Glass Interface in Bioactive Glass Coatings on Ti-6Al-4V", Journal of Materials Science: Materials in Medicine 12 ( 2001 ) 413.
29. S. J. Ding, C. P. Ju and J. H. Chern-Lin, "Morphology and Immersion Behaviour of Plasma-Sprayed Hydroxyapatite/Bioactive Glass Coatings", Journal of Materials Science: Materials in Medicine 11 ( 2000 ) 183.
30. D. B. Chrisey, G. K. Hubler ( eds ); "Pulsed Laser Deposition of Thin Films", John Wiley & Sons, New York, 1994.
31. A. Merolli, A. Cacchioli, L. Gianotta, P. Tranquilli-Leali, "Energy Dispersive Analysis ( EDX ) of a Degradable Bioactive-Glass Coating on Ti6Al4V in-vivo", Journal of Materials Science: Materials in Medicine 12 (2001) 727.
32. P. González, J. Serra, S. Liste, S. Chiussi, B. León, M. Pérez-Amor, "Ageing of Pulsed Laser Deposited Bioactive Glass Films", Vacuum (2001) in press.

### EXPLANATION OF THE INVENTION

The object of this invention is to develop a new biocompatible material to be used in medical implants. The material comprises a biomorphic ceramic base that is obtained from vegetable precursors that are coated with bioactive glass.
A. The procedure that is followed to obtain the ceramic material is patented [2] and consists of the following:
   1 - Drying the wood
      In a stove for one day at 70 °C, in the case of wood that has already been prepared for industrial use. In the case of recently cut wood, it is first immersed in alcohol and its drying period is increased to three days.
   2 - Pyrolysis process
      This is carried out at a heating rate that ranges from 0.5 to 2 °C per minute until it reaches temperatures greater than 600 °C. As soon as the maximum temperature has been reached it is cooled at a rate ranging from 1 to 5 °C. This process will be carried out at partial pressures of oxygen of less than 10⁻⁴ Torr.
   3 - Infiltration process
      This is carried out with very pure silicon (preferably ground monocrystalline silicon ).
      The silicon is placed in solid form in zones that are conducive to its later infiltration in the preform of carbon, helped by capillarity through the pores.
      The silicon and the preform are placed on a non-reactive crucible, for example one that is made of boron nitride.
      The amount of silicon will be calculated by means of the atomic weights and stequiometry, an excess quantity of 20% being added so that the carbon reacts completely and no silicon remains on the surface of the sample. This point is very important, because this ensures that the final finish for the piece is not necessary.
      The infiltration temperature will range from 1,410 °C to 1,600 °C. The infiltration time will be established on the basis of the size and the shape of the preform that has to be infiltrated. The process will be carried out at pressures of less than 10⁻³ Torr.
      After the infiltration process has been completed, the silicon that is left inside the sample can be removed by putting it in contact with another carbon preform and treating the whole thermally in a vacuum at a temperature higher than 1,410 °C.
B. Once the ceramic material has been obtained the procedure shown below is followed:
   1 - Cleaning the biomorphic ceramic to be coated.
      This is done with isopropanol in an ultrasonic bath for three minutes, followed by drying.
   2 - Inserting the ceramic material into an ablation chamber that is equipped with a high vacuum system, a heater for the substrate, a rotating target carrier and regulation of the flow rate and pressure of gases in the chamber;
   3 - Placing a piece of bioactive glass belonging to the SiO₂-Na₂O-K₂O-B₂O₃-MgO-CaO-P₂O₅ system on the rotating target carrier, this being used as the ablation target;
   4 - Carrying out a prior vacuuming to pressures of 10⁻⁵ mbar.
   5 - Focusing the laser and the deposition
      Irradiation of the glass target with ultraviolet radiation ( λ = 193 nm ) from an ArF laser; the laser beam is focused with the aid of a lens of melted silicon and is inserted in the chamber through a window that is transparent to this radiation; this stage can also be carried out in the presence either of an atmosphere of inert gas ( argon, nitrogen or others ) or a reactive gas ( steam, oxygen, N₂O or others ): when the material is being ablated this gives rise to the formation of an ablation plume in which the resulting products are transferred to the biomorphic SiC substrate, coating it with a layer of bioactive glass;

### REALISATION OF THE INVENTION

By way of example, an explanation is given of a process for using this biocompatible material to make a screw for dental applications. With a view to this, a substrate of biomorphic ceramics is manufactured using vegetable precursors that are then covered with bioactive glass that is deposited by means of pulsed laser ablation.

The biomorphic ceramic substrate manufacturing process consists of the following two stages, according to the silicon carbide ceramic manufacturing procedure that is described in Patent ES 2187371:
1 - Selecting the vegetable precursor
   A cylinder made of commercial beech wood 11 mm long and 4 mm in diameter is taken. The weight of this piece of wood is 0.095 grams ( density 0.69 g/cm³ ) before any process is initiated with it.
2 - Pyrolysis
   The piece of wood is placed in the centre of a ceramic tube made of aluminium oxide, through which argon ( an inert gas ) flows at a pressure slightly greater than atmospheric pressure. The tube passes through the centre of a furnace. The ends of the ceramic tube are cooled so that the rubber joints at these ends do not melt.
   After a few minutes have elapsed and the argon is flowing through the ceramic tube in a stable way, the system is heated. It is heated at a rate of 0.4 °C per minute until the temperature reaches 1,000 °C ( the heating process lasts 41 hours and 40 minutes ). The temperature is kept at 1,000 °C for 30 minutes, and then it is cooled at a rate of 5 °C per minute ( the cooling time is 3 hours and 20 minutes ).
   This process turns the piece of wood into carbon. The piece of carbon is now 8.10 mm long and has a diameter of 2.95 mm. The piece of carbon weighs 0.027 grams (density 0.49 g/cm³).
3 - Modelling the carbon
   The piece is given a finish that involves marking the striated forms on the carbon cylinder.
4 - Infiltration
   0.090 grams of monocrystalline silicon are placed on the piece of carbon and it is placed in a crucible whose walls have been covered with boron nitride. The crucible is put into a tube furnace, which is then vacuumed with the aid of a rotary pump.
   The system is heated at a rate of 10 °C per minute until it reaches a temperature of 1,550 °C ( the heating time is 2 hours and 25 minutes ). The temperature is kept at 1,550 °C for 30 minutes, and then it is cooled at a rate of 10 °C per minute until it is at room temperature ( the cooling time is 2 hours and 25 minutes ).
   As soon as the system reaches room temperature the rotary pump is switched off, air is forced into the tube, and the crucible is extracted.
   The sample of biomorphic ceramic piece that is obtained as an end product does not change its dimensions within the error of measurement and it weighs 0.0925 grams.

Once the biomorphic ceramic piece is obtained, the process of coating with bioactive glass begins with pulsed laser ablation. The coating process consists of the following steps:
1 - Cleaning the biomorphic ceramic piece that has to be coated
   This is done with isopropanol in an ultrasonic bath for three minutes, followed by drying.
2 - Placing a piece of bioactive glass belonging to the SiO₂-Na₂O-K₂O-B₂O₃-MgO-CaO-P₂O₅ system on the rotating target carrier, this being used as the ablation target;
   Rotating speed of the target = 3 r.p.m.
3 - Carrying out a vacuuming
   Pressure = 10⁻³ mbar.
4 - Focusing the laser and the deposition
   Irradiation of the glass target with ultraviolet radiation ( λ = 193 nm ) from an ArF laser; the laser beam is focused with the aid of a lens of melted silicon and is inserted in the chamber through a window that is transparent to this radiation;
   Temperature of the substrate = 200 °C
   Energy of the laser = 175 mJ/pulse
   Repeating frequency of the laser pulse = 10 Hz

When the material is being ablated this gives rise to the formation of an ablation plume in which the resulting products are transferred to the biomorphic SiC substrate, coating it with a layer of bioactive glass;

## Claims

1. Process for the production of a biocompatible material coating a biomorphic ceramic base, *obtained* from vegetable precursors, with bioactive glass using a pulsed laser ArF, using the following steps:
1) Cleaning the biomorphic ceramic piece that has to be coated with isopropanol in an ultrasonic bath for three minutes, followed by drying.
2) Inserting the ceramic material into an ablation chamber that is equipped with a high vacuum system, a heater for the substrate, a rotating target carrier and regulation of the flow rate and pressure of gases in the chamber;
3) Placing a piece of bioactive glass belonging to the SiO₂-Na₂O-K₂O-B₂O₃- MgO-CaO-P₂O₅ system on the rotating target carrier, this being used as the ablation target;
4) Carrying out a prior vacuuming to pressures of 10⁻⁵ mbar or creating in the ablation chamber an atmosphere of inert gas such as argon, nitrogen or others, or a reactive gas such as steam, oxygen, N₂O or others, and irradiation of the glass target with ultraviolet radiation ( λ = 193 nm ) from an ArF laser; the laser beam being focused with the aid of a lens of melted silicon and inserted in the chamber through a window that is transparent to this radiation;
5) When ablating the material thus giveing rise to the formation of an ablation plume in which the resulting products are transferred to the biomorphic SiC substrate, coating it with a layer ofbioactive glass.

2. Process for the production of a biocompatible material obtained according with Claim 1, **characterised by** the fact that the biocompatible material is adapted for being used in medical implants.

## Patentansprüche

1. Verfahren zur Herstellung eines biokompatiblen Werkstoffs, der eine biomorphe Keramikgrundlage beschichtet, der aus pflanzlichen Vorläufern erzielt wurde, mit bioaktivem Glas unter Einsatz eines gepulsten ArF-Lasers, wobei in folgenden Schritten vorgegangen wird:
1) Dreiminütige Reinigung des zu beschichtenden biomorphen Keramikteils mit Isopropanol in einem Ultraschallbad und nachfolgende Trocknung.
2) Einbringen des keramischen Werkstoffs in eine Ablationskammer, die mit einem Hochvakuumsystem, einem Heizgerät für das Substrat, einem drehenden Targetträger und Regelung des Durchsatzes und Drucks der Gase in der Kammer ausgestattet ist;
3) Aufbringen eines Stücks des bioaktiven Glases, das zu dem SiO₂-Na₂O-K₂O-B₂O₃-MgO-CaO-P₂O₅ System gehört, auf den drehenden Targetträger, das als Ablationstarget verwendet wird;
4) Vornahme einer vorherigen Absaugung mit Drücken von 10⁻⁵ mbar oder in der Ablationskammer Schaffung einer Atmosphäre mit inertem Gas wie Argon, Stickstoff oder anderen oder einem reaktiven Gas wie Wasserdampf, Sauerstoff, N₂O oder anderen und Bestrahlung des Glastargets mit ultravioletter Strahlung (λ= 193 nm) von einem ArF-Laser, wobei der Laser mit Hilfe einer Linse aus geschmolzenem Silikon fokussiert und über ein für diese Strahlung transparentes Fenster in die Kammer gebracht wird;
5) Beim Abtragen des Materials bildet sich eine Ablationswolke, wobei die entstehenden Produkte auf das biomorphe SiC-Substrat übertragen werden und es mit einer Schicht biokativen Glases beschichten;

2. Verfahren zur Herstellung eines biokompatiblen Werkstoffs, der nach Patentanspruch 1 erzielt wird, das sich **dadurch kennzeichnet, dass** der biokompatible Werkstoff auf den Einsatz in medizinischen Implantationen angepasst wird.

## Revendications

1. Procédé de production d'un matériau biocompatible recouvrant une base biomorphique en céramique, obtenu à partir de précurseurs végétaux, avec du verre bioactif en utilisant un laser à impulsions ArF, en utilisant les étapes suivantes :
1) Nettoyage de la pièce biomorphique en céramique qui doit être recouverte d'isopropanol dans un bain à ultrasons pendant trois minutes, suivi de séchage.
2) Insertion du matériau céramique dans une chambre d'ablation qui est équipée d'un système à vide élevé, d'un réchauffeur de substrat, d'un porteur de cibles rotatif et d'une régulation du débit et de la pression des gaz dans la chambre.
3) Insertion d'une pièce en verre bioactif appartenant au système SiO₂-Na₂O-K₂O-B₂O₃-MgO-CaO-P₂O₅ sur le porteur de cibles rotatif, ceci étant utilisé comme la cible d'ablation.
4) Application d'un vide préalable à des pressions de 10⁻⁵ mbar ou création dans la chambre d'ablation d'une atmosphère de gaz inerte tel que l'argon, l'azote ou autres ou de gaz réactif tel que vapeur, oxygène, N₂O ou autres, et irradiation de la cible en verre avec un rayonnement ultraviolet (λ = 193 nm) provenant d'un laser ArF ; le rayon du laser étant mis au point à l'aide d'une lentille en silicone coulé et pénétrant dans la chambre par une fenêtre qui est transparente à cette radiation.
5) Quand l'ablation du matériau a lieu, elle donne naissance à la formation d'un panache d'ablation dans lequel les produits résultants sont transférés vers le substrat SiC biomorphique, le recouvrant d'une couche de verre bioactif

2. Procédé de production d'un matériau biocompatible obtenu conformément à la revendication 1, **caractérisé par le fait que** le matériau biocompatible est adopté pour être utilisé dans les implants médicaux.
